# EUROPEAN PATENT SPECIFICATION

(11) **EP 1 756 270 B1**
(45) Date of publication and mention of the grant of the patent: **03.03.2010**
(21) Application number: 05763889.2
(22) Date of filing: 06.04.2005
(51) Int. Cl.: C07K 14/435, G01N 33/574, A61K 38/17, A61K 31/4188, C12N 5/071, C12Q 1/00, A61K 39/00, C07K 1/00, A61P 35/00

(54) **DIAGNOSIS AND TREATMENT OF MYELOID AND LYMPHOID CELL CANCERS WITH CHLOROTOXIN OR A DERIVATIVE**
DIAGNOSE UND BEHANDLUNG VON MYELOID- UND LYMPHOIDZELLENKREBS MIT CHLOROTOXIN ODER DERIVAT
DIAGNOSTIC ET TRAITEMENT DES CANCERS DE CELLULES MYELOIDES ET LYMPHOIDES AVEC CHLOROTOXINE OU SON DERIVE

(30) Priority: 06.04.2004 US 559433 P
(43) Date of publication of application: 28.02.2007
(60) Divisional application: 09167183.4
(73) Proprietor: Transmolecular, Inc., Birmingham, AL 35243 (US)
(72) Inventor: ALVAREZ, Vernon, L., Birmingham, AL 35243 (US); GONDA, Matthew, A., Birmingham, AL 35242 (US)
(74) Representative: Care, Alison
(86) International application number: PCT/US2005/011523
(87) International publication number: WO 2005/099774

(56) References cited:
- WO-A1-03/101474
- WO-A1-03/101475
- WO-A2-03/000203
- LYONS S A ET AL: "CHLOROTOXIN, A SCORPION-DERIVED PEPTIDE, SPECIFICALLY BINDS TO GLIOMAS AND TUMORS OF NEUROECTODERMAL ORIGIN" GLIA, WILEY-LISS, NEW YORK, NY, US, vol. 39, no. 2, August 2002 (2002-08), pages 162-173, XP009006886 ISSN: 0894-1491
- DESHANE JESSY ET AL: "Chlorotoxin inhibits glioma cell invasion via matrix metalloproteinase-2" JOURNAL OF BIOLOGICAL CHEMISTRY, AMERICAN SOCIETY OF BIOLOCHEMICAL BIOLOGISTS, BIRMINGHAM,, US, vol. 278, no. 6, 25 November 2002 (2002-11-25), pages 4135-4144, XP002398052 ISSN: 0021-9258

## Description

### Field of the Invention

The invention relates to the treatment of myeloproliferative and lymphoproliferative disorders with chlorotoxin or derivatives thereof.

### Background of the Invention

Blood cells, in general, are specified as being either lymphoid (*e.g*., lymphocytes, etc.) or myeloid (*e.g.*, granulocytes, monocytes, erythrocytes and platelets). Accordingly, hematological malignancies are organized into lymphoproliferative or myeloproliferative disorders. Each of these disorders is operationally classified as being acute or chronic, depending upon the proportion of immature precursor cells (blasts) in the bone marrow. In the myeloid lineage, the presence of more than thirty percent blasts in the bone marrow defines acute myeloid leukemia. A myeloid disorder that is not acute myeloid leukemia is referred to as either a myelodysplastic syndrome or a chronic myeloproliferative disease based, respectively, on the presence or absence of trilineage morphologic displasia, primarily involving red blood cells. The chronic myeloproliferative diseases include chronic myelogenous leukemia (CML), essential thrombocythemia, polycythemia vera and agnogenic myeloid metaplasia. Occasionally, a chronic myeloid disorder is not classifiable as either myelodysplastic syndrome or chronic myeloproliferative disease. Examples include atypical CML and chronic neutrophilic leukemia.

As a group, the chronic myeloproliferative diseases are interrelated in that the clonal process originates at the myeloid progenitor cell level and may, secondarily, cause marrow fibrosis or undergo leukemic transformation. Among the chronic myeloproliferative diseases, only CML has been biologically characterized by the presence of a reciprocal genetic translocation between chromosomes nine and twenty-two. A similar consistent genetic abnormality has not been associated with the other chronic myeloproliferative diseases, and clinical diagnosis is base on the presence or absence of certain clinical and laboratory characteristics. An increased red blood cell mass is required for the diagnosis of polycythemia vera. Similarly, the presence of substantial bone marrow fibrosis not associated with CML or polycythemia vera is the hallmark of agnogenic myeloid metaplasia. The diagnosis of essential thrombocythemia is one of exclusion, representing clonal thrombocytosis that is not classifiable as agnogenic myeloid metaplasia, polycythemia vera, myelodysplastic syndrome or CML.

Lymphoproliferative disorders can be divided into two major categories, Non-Hodgkin's lymphoma encompassing over twenty discrete entities (see Table 1) and Hodgkin's lymphoma, which is localized to the lymph nodes. Currently, immunohistochemistry is used to differentiate between Hodgkin's and non-Hodgkin's lymphomas. In most cases of Hodgkin's disease, a particular cell known as the Reed-Sternberg cell is found in the biopsies. This cell is not usually found in other lymphomas, so these are designated non-Hodgkin's lymphoma. The non-Hodgkin's lymphomas (NHL) are a collection of lymphoid malignancies with a diverse pathology and natural history. This diversity is illustrated by the different histologic subtypes and classifications of NHL that have appeared over the years. With the rapid progress in our understanding of the biology of lymphomas, new systems of classification have better described this group of diseases.

**Table 1. Types of Non-Hodgkin's Lymphoma**

| **B cell neoplasms** | |
|---|---|
| Precursor B-cell lymphoblastic leukemia/lymphoma | |
| Mature | B cell neoplasms |
| | B cell chronic lymphocytic leukemia/small lymphocytic lymphoma |
| | B cell prolymphocytic leukemia |
| | Lymphoplasmacytic lymphoma |
| | Splenic marginal zone B cell lymphoma |
| | Hairy cell leukemia |
| | Extranodal marginal zone B cell lymphoma |
| | Mantle cell lymphoma |
| | Follicular lymphoma |
| | Nodal marginal zone lymphoma |
| | Diffuse large B cell lymphoma |
| | Burkitt's lymphoma |
| | Plasmacytoma |
| | Plasma cell myeloma |

| **T cell neoplasms** | |
|---|---|
| | T cell prolymphocytic leukemia |
| | T cell large granular lymphcytic leukemia |
| | NK cell leukemia |
| | Extranodal NK/T cell lymphoma |
| | Mycosis fungoides |
| | Primary cutaneous anaplastic large cell lymphoma |
| | Subcutaneous panniculitis-like T cell lymphoma |
| | Enteropathy-type intestinal T cell lymphoma |
| | Hepatosplenic gamma-delta T cell lymphoma |
| | Angioimmunoblastic T cell lymphoma |
| | Peripheral T cell lymphoma |
| | Anaplastic large cell lymphoma |
| | Adult T cell lymphoma |

Although significant advances have been made in the treatment of non-Hodgkin's lymphoma over the past two decades, a curative regimen for patients with B and T cell lymphomas has yet to be developed. In addition, durable remission in patients treated with various regimens for refractory intermediate- and high-grade lymphomas have been relatively rare (Klimo (1988) Chemotherapy for aggressive non-Hodgkin's lymphomas, In DeVita et al. (eds), Cancer: Principles and Practice of Oncology, Lippincott, 1-12). Recent attempts utilizing supralethal chemotherapy combined with radiotherapy followed by bone marrow transplantation have resulted in an approximately twenty percent long term disease-free survival rate (Applebaum et al. (1987) J. Clin. Oncol. 5, 1340-1341). However, most patients treated in this manner die of lymphoma or treatment related complications. Therefore, new strategies for the treatment of non-Hodgkin's lymphomas are needed. These strategies should have as their goal the maximization of therapeutic effect coupled with the minimization of toxicity.

One approach involves the use of monoclonal antibodies which recognize tumor-associated antigens as a means of targeting drugs or radioisotopes to tumor cells. This approach is particularly attractive in the case of non-Hodgkin's lymphomas as the tumor cells of these lymphomas display a variety of tumor-restricted antigens on their cell surfaces which would be available for targeting (McMichael (1987) Leukocyte Typing III, Oxford University Press, 302-363 and 432-469). Of all the malignancies that have been treated with monoclonal antibodies to date, the lymphomas have yielded the most dramatic results. Most of the tumor responses, however, have been incomplete and of relatively short duration. Because of these limited results, there exists a need for targeting agents other than antibodies that will be useful for the detection and treatment of lymphoma.

Chlorotoxin is a thirty-six amino acid protein naturally derived from *leiurus quinquestriatus* scorpion venom (DeBin et al. (1993) Am. J. Physiol. 264: C361-369). Compositions (see U.S. Patents 5,905,027 and 6,429,187) and methods (see U.S. Patents 6,028,174 and 6,319,891 and WO 03/101474) for diagnosing and treating neuroectodermal tumors (*e.g*., gliomas and meningiomas) and prostate cancer have been developed based on the ability of chlorotoxin to bind to tumor cells of neuroectodermal origin (Soroceanu et al. (1998) Cancer Res. 58, 4871-4879; Ullrich et al. (1996) Neuroreport 7,1020-1024; Ullrich et al. (1996) Am. J. Physiol. 270, C1511-C1521). Diagnosis of neuroectodermal tumors is accomplished by identification of labeled chlorotoxin bound to tumor cells while treatment of neuroectodermal tumors is accomplished by targeting tumors with cytotoxic agents linked to chlorotoxin. The present invention expands this area of therapeutics by providing a composition for treating myeloproliferative and lymphoproliferative disorders based on the finding that chlorotoxin and derivatives thereof bind to myeloid and lymphoid cancer cells.

### Summary of the Invention

The invention encompasses a method of detecting the presence of a lymphoproliferative or myeloproliferative disorder in a mammal comprising (a) contacting a biological sample isolated from the mammal (SEQ ID NO:1) with a composition comprising chlorotoxin or a fragment thereof having the amino acid sequence KGRGKSY (SEQ ID NO:8), and (b) detecting the presence of binding of the composition to the sample, wherein the presence of binding indicates the presence of a lymphoproliferative or myeloproliferative disorder in the mammal.

The invention also includes a composition comprising chlorotoxin (SEQ NO:1) or a fragment thereof having the amino acid sequence KGRGKSY (SEQ ID NO:8) for use in the diagnosis of a lymphoproliferative or myeloproliferative disorder in a mammal, wherein the composition is administered to the mammal and (b) detection of binding of the composition in the mammal indicates the presence of a lymphoproliferative or myeloproliferative disorder in the mammal.

The invention includes a composition comprising chlorotoxin (SEQ ID NO:1) or a fragment thereof having the amino acid sequence KGRGKSY (SEQ ID NO:8) for use in treating a lymphoproliferative or myeloproliferative disorder in a mammal wherein the chlorotoxin or fragment thereof is linked to a cytotoxic agent. In some embodiments, the mammal is a human.

The invention includes the use of a composition comprising chlorotoxin (SEQ ID NO:1) or a fragment thereof having the amino acid sequence KGRGKSY (SEQ ID NO:8) in the manufacture of a medicament for treating a lymphoproliferative or myeloproliferative disorder in a mammal, wherein the chlorotoxin or fragment thereof is linked to a cytotoxic agent.

The lymphoproliferative disorders may be a non-Hodgkin's lymphoma, and includes instances where the non-Hodgkin's lymphoma is a B cell neoplasm and the B cell neoplasm is a Precursor B cell lymphoblastic leukemia/lymphoma or a mature B cell neoplasm. Examples of mature B cell neoplasm encompassed in the methods of the invention include, but are not limited to, B cell chronic lymphocytic leukemia/small lymphocytic lymphoma, B cell prolymphocytic leukemia, Lymphoplasmacytic lymphoma, Splenic marginal zone B cell lymphoma, Hairy cell leukemia, Extranodal marginal zone B cell lymphoma, Mantle cell lymphoma, Follicular lymphoma, Nodal marginal zone lymphoma, Diffuse large B cell lymphoma, Burkitt's lymphoma, Plasmacytoma, and Plasma cell myeloma.

The non-Hodgkin's lymphoma may be a T cell neoplasm. Examples of T cell neoplasms include, but are not limited to, T cell prolymphocytic leukemia, T cell large granular lymphcytic leukemia, NK cell leukemia, Extranodal NK/T cell lymphoma, Mycosis fungoides. Primary cutaneous anaplastic large cell lymphoma, Subcutaneous panniculitis-like T cell lymphoma, Enteropathy-type intestinal T cell lymphoma, Hepatosplenic gamma-delta T cell lymphoma, Angioimmunoblastic T cell lymphoma, Peripheral T cell lymphoma, Anaplastic large cell lymphoma and Adult T cell lymphoma.

The myeloproliferative disorder may be selected from the group consisting of polycythemia vera (PV), essential thrombocythemia (ET), agnogenic myeloid metaplasia (AMM) also referred to as idiopathic myelofibrosis (IMF) and chronic myelogenous leukemia (CML).

Where the invention relates to diagnosis, the chlorotoxin or a derivative thereof may be linked to a second polypeptide. The second polypeptide can comprise a binding domain which binds specifically of an epitope expressed only by a myeloid or lymphoid cancer cell and may be an antibody or fragment thereof and/or a stabilization domain which prevent degradation of the fusion polypeptide. Examples of stabilization domains include, but are not limited to, polyhistidine and human serum albumin. Where the invention relates a treatment, the chlorotoxin or a derivative thereof is linked to a cytotoxic agent. Examples of cytotoxic agents include, but are not limited to, gelonin, ricin, saponin, pseudonomas exotoxin, pokeweed antiviral protein, diphtheria toxin and complement proteins. In the invention, chlorotoxin or a derivative thereof may be labeled and the label may be ¹³¹I. The amount of chlorotoxin administered in the invention can comprise between about 0.01 µg/kg body weight to about 2.0 mg/kg body weight.

### Brief Description of the Drawings

Figure 1 depicts the results of FACS analysis of lymphoid cancer cells (B lymphocytes (Raji) and B lymphoblasts (Daudi) from Burkitt's lymphoma) treated with chlorotoxine or a peptide fragment of chlorotoxin previously identified as the binding domain (peptide 21 (SEQ ID NO: 10) and peptide 8 (SEQ ID NO: 8)).
Figure 2 depicts the results of FACS analysis of lymphoid cancer cells (T lymphoblasts (Molt-4) from acute lymphoblastic leukemia) treated with chlorotoxin or a peptide fragment of chlorotoxin previously identified as the binding domain (peptide 21 (SEQ ID NO: 10) and peptide 8 (SEQ ID NO: 8)).
Figure 3 depicts the results of experiments demonstrating the dose-dependent synergistic effects of chlorotoxin in combination with doxorubicin on Raji cell proliferation.
Figure 4 depicts the results of experiments demonstrating the dose-dependent synergistic effects of chlorotoxin in combination with doxorubicin on Daudi cell proliferation.

### Detailed Description of the Invention

It has been determined that chlorotoxin and the fragment thereof having the amino acid sequence KGRGKSY specifically and selectively bind to myeloid and lymphoid cancer cells. The present invention therefore includes the diagnosis and treatment of myleoproliferative and lympoproliferative diseases by contacting the myeloid or lymphoid cancer cell with an effective amount of chlorotoxin or fragment thereof.

Inhibition or arrest of cell proliferation associated with a myleoproliferative or lympoproliferative disease, can serve to enhance natural defenses within the subject For example, arresting or inhibiting the growth of cancer cells enhances the ability of the immune system to mount a more effective response to the cancer.

### Myleoproliferative or Lympoproliferative Disease

As used herein, the term "myeloproliferative disease" unless otherwise indicated, refers to myeloid cell growth that is independent of normal regulatory mechanisms. This includes the abnormal growth and/or proliferation of myeloid cells in both benign and malignant states of neoplastic diseases. Inhibition of abnormal cell growth can occur by a variety of mechanisms including, but not limited to, cell death, apoptosis, arrest of mitosis, inhibition of cell division, transcription, translation, transduction, etc. Examples of myeloproliferative diseases include, but are not limited to, polycythemia vera (PV), essential thrombocythemia (ET), agnogenic myeloid metaplasia (AMM) also referred to as idiopathic myelofibrosis (IMF) and chronic myelogenous leukemia (CML).

As used herein, the term "lymphoproliferative disease" unless otherwise indicated, refers to lymphoid cell growth that is independent of normal regulatory mechanisms. This includes the abnormal growth and/or proliferation of lymphoid cells in both benign and malignant states of neoplastic diseases. Inhibition of abnormal cell growth can occur by a variety of mechanisms including, but not limited to, cell death, apoptosis, arrest of mitosis, inhibition of cell division, transcription, translation, transduction, etc.

In one embodiment, the lymphoproliferative disorder is non-Hodgkin's lymphoma. In another embodiment, the non-Hodgkin's lymphoma is a B cell neoplasm selected from Precursor B-cell lymphoblastic leukemia/lymphoma or a mature B cell neoplasm. Mature B cell neoplasms include, but are not limited to, B cell chronic lymphocytic leukemia/small lymphocytic lymphoma, B cell prolymphocytic leukemia, Lymphoplasmacytic lymphoma, Splenic marginal zone B cell lymphoma, Hairy cell leukemia, Extranodal marginal zone B cell lymphoma, Mantle cell lymphoma, Follicular lymphoma, Nodal marginal zone lymphoma, Diffuse large B cell lymphoma, Burkitt's lymphoma, Plasmacytoma, and Plasma cell myeloma.

The invention also encompasses treatment of non-Hodgkin's lymphoma which is a T cell neoplasm. Exemplary T cell neoplasms, include, but axe not limited to T cell prolymphocytic leukemia, T cell large granular lymphcytic leukemia, NK cell leukemia, Extranodal NK/T cell lymphoma, Mycosis fungoides, Primary cutaneous anaplastic large cell lymphoma, Subcutaneous panniculitis-like T cell lymphoma, Enteropathy-type intestinal T cell lymphoma, Hepatosplenic gamma-delta T cell lymphoma, Angioimmunoblastic T cell lymphoma, Peripheral T cell lymphoma, Anaplastic large cell lymphoma and Adult T cell lymphoma.

In one embodiment of the invention, the myleoproliferative or lympoproliferative disease is cancer. As used herein, the term "cancer" unless otherwise indicated, refers to diseases that are characterized by uncontrolled, abnormal cell growth and/or proliferation. Types of cancer where the compositions are useful include, but are not limited to, cancers associated with myeloid and/or lymphoid stem cell transformation.

As used herein, the term "chlorotoxin" unless otherwise described, refers to the full-length, thirty-six amino acid polypeptide naturally derived from *Leiurus quinquestriatus* scorpion venom (DeBin et al. (1993) Am. J. Physiol. 264, C361-369) which comprises the amino acid sequence of native chlorotoxin as set forth in SEQ ID NO: 1. The term "chlorotoxin" includes polypeptides comprising SEQ ID NO: 1 which have been synthetically or recombinantly produced, such as those disclosed in U.S. Patent 6,319,891.

As used herein, the term "chlototoxin subunit" or "subunit of chlorotoxin" refers to a peptide comprising less than thirty-six contiguous amino acids of chlorotoxin and which is capable of specifically binding to cancer cells.

Chlorotoxin and peptide derivatives thereof can be prepared using standard solid phase (or solution phase) peptide synthesis methods, as is known in the art. In addition, the nucleic acids encoding these peptides may be synthesized using commercially available oligonucleotide synthesis instrumentation and produced recombinantly using standard recombinant production systems. The production using solid phase peptide synthesis is necessitated if non-gene-encoded amino acids are to be included.

Fragments of chlorotoxin useful in the invention comprise the amino acid sequence KGRGKSY (SEQ ID NO: 8), corresponding to amino acid residues 23 to 29 of SEQ ID NO: 1.

Disclosed herein are corresponding polypeptide toxins of other scorpion species, also generally referred to herein as chlorotoxin derivatives, that display similar or related activity to chlorotoxin.

As used herein, the term "related scorpion toxin" refers to any of the toxins or related peptides, such as those disclosed in Table 1, displaying amino acid and/or nucleotide sequence identity to chlorotoxin. Examples of related scorpion toxins include, but are not limited to, CT neurotoxin from *Mesobuthus martensii* (GenBank Accession AAD47373), Neurotoxin BmK 41-2 from *Buthus martensii karsch* (GenBank Accession A59356), Neurotoxin Bm12-b from *Buthus martensii* (GenBank Accession AAK16444), Probable Toxin LQH 8/6 from *Leiurus quinquestriatus hebraeu* (GenBank Accession P55966), Small toxin from *Mesobuthus tamulus sindicus* (GenBank Accession P15229).

Homology or sequence identity at the nucleotide or amino acid sequence level is determined by **BLAST** (Basic Local Alignment Search Tool) analysis using the algorithm employed by the programs **blastp, blastn, blastx, tblastn** and **tblastx** (Altschul et al. (1997) Nucleic Acids Res. 25, 3389-3402 and Karlin et al. (1990) Proc. Natl. Acad. Sci. USA 87, 2264-2268 which are tailored for sequence similarity searching. The approach used by the **BLAST** program is to first consider similar segments, with gaps (non-contiguous) and without gaps (contiguous), between a query sequence and a database sequence, then to evaluate the statistical significance of all matches that are identified and finally to summarize only those matches which satisfy a preselected threshold of significance. For a discussion of basic issues in similarity searching of sequence databases, see Altschul et al. (1994) Nature Genetics 6, 119-129. The search parameters for histogram, descriptions, alignments, expect (*i.e*., the statistical significance threshold for reporting matches against database sequences), cutoff, matrix and filter (low complexity) are at the default settings. The default scoring matrix used by blastp, blastx, tblastn, and tblastx is the **BLOSUM62** matrix (Henikoff et al. (1992) Proc. Natl. Acad. Sci. USA 89, 10915-10919), recommended for query sequences over eighty-five nucleotides or amino acids in length.

For **blastn,** the scoring matrix is set by the ratios of **M** (*i.e*., the reward score for a pair of matching residues) to N (*i.e*., the penalty score for mismatching residues), wherein the default values for **M** and **N** are +5 and -4, respectively. Four **blastn** parameters were adjusted as follows: Q=10 (gap creation penalty); R=10 (gap extension penalty); wink=1 (generates word hits at every wink^{th} position along the query); and gapw=16 (sets the window width within which gapped alignments are generated). The equivalent **Blastp** parameter settings were Q=9; R=2; Wink=1; and gapw=32. A **Bestfit** comparison between sequences, available in the GCG package version 10.0, uses DNA parameters GAP=50 (gap creation penalty) and LEN=3 (gap extension penalty) and the equivalent settings in protein comparisons are GAP=8 and LEN=2.

Fusion proteins, or N-terminal or C-terminal extensions, into the peptide sequence shall not be construed as affecting homology. Examples of such extensions include, but are not limited to, the following sequences:
HHHHHHMCMPCFTTDHQMARKCDDCCGGKGRGKCYGPQCLCR (SEQ ID NO: 2),
YMCMPCFTTDHQMARKCDDCCGGKGRGKCYGPQCLCR (SEQ ID NO: 3),
YSYMCMPCFTTDHQMARKCDDCCGGKGRGKCYGPQCLCR (SEQ ID NO: 4).

Contemplated peptide variants further include but are not limited to the following sequences:
MCMPCFTTDHQMARKCDDCCGGKGRGKCFGPQCLCR (SEQ ID NO: 5),
RCKPCFTTDPQMSKKCADCCGGKGKGKCYGPQCLC (SEQ ID NO: 6),
RCSPCFTTDQQMTKKCYDCCGGKGKGKCYGPQCICAPY (SEQ ID NO: 7).

The invention can be utilized *in vivo,* ordinarily in mammals, such as humans, sheep, horses, cattle, pigs, dogs, cats, rats and mice or *in vitro.* The invention is particularly useful in the treatment of human subjects.

### Recombinant Proteins and Polypeptides

The present invention further provides for the use of recombinant chlorotoxin protein or fragment thereof for treatment of myeloproliferative or lymphoproliferative disorders. Nucleic acid molecules that encode chlorotoxin or fragment thereof can be used to recombinantly produce these polypeptide. Such nucleic acid molecules can be in an isolated form, or can be operably linked to expression control elements or vector sequences. Host cells can contain the vectors via transformation, transfection, electroporation or any other art recognized means of introducing a nucleic acid into a cell.

As used herein, a "replicon" is any genetic element (*e.g*., plasmid, chromosome, virus) that functions as an autonomous unit of DNA replication *in vivo* (*i.e*., capable of replication under its own control).

As used herein, a "vector" is a replicon, such as plasmid, phage or cosmid, to which another nucleic acid *(e.g.,* DNA) segment may be attached so as to bring about the replication of the attached segment. Vectors of the invention include viral vectors.

As used herein, a "nucleic acid" refers to the polymeric form of ribonucleotide or deoxyribonucleotides (adenine, guanine, thymine, and/or cytosine) in either its single stranded form, or in double-stranded helix. This term refers only to the primary and secondary structure of the molecule and is not limited to any particular tertiary form. Thus, this term includes single-stranded RNA or DNA, double-stranded DNA found in linear DNA molecules (*e.g*., restriction fragments), viruses, plasmids, and chromosomes. In discussing the structure of particular double-stranded DNA molecules, sequences may be described herein according to the normal convention of giving only the sequence in the 5' to 3' direction along the non-transcribed strand of DNA (*e.g.,* the strand having a sequence homologous to the mRNA).

A nucleic acid "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxy) terminus. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

As used herein, a "promoter sequence" is a DNA regulatory region capable of binding RNA polymerase in a cell and initiating transcription of a downstream (3' direction) coding sequence. For purposes of defining the present invention, the promoter sequence is bounded (inclusively) at its 3' terminus by the transcription initiation site and extends upstream (5' direction) to include the minimum number of bases or elements necessary to initiate transcription at levels detectable above background. Within the promoter sequence will be found a transcription initiation site, as well as protein binding domains responsible for the binding of RNA polymerase. Eukaryotic promoters will often, but not always, contain "TATA" boxes and "CAT" boxes.

A coding sequence is "under the control" of transcriptional and translational control sequences in a cell when RNA polymerase transcribes the coding sequence into mRNA, which is then translated into the protein encoded by the coding sequence.

A "signal sequence" can be included before the coding sequence or a signal peptide sequence from a scorpion toxin may be used. This sequence encodes a signal peptide, N-terminal to the polypeptide, that communicates to the host cell to direct the polypeptide to the cell surface or secrete the polypeptide into the media. This signal peptide is clipped off by the host cell before the protein leaves the cell. Signal sequences can be found associated with a variety of proteins native to prokaryotes and eukaryotes. For instance, alpha-factor, a native yeast protein, is secreted from yeast, and its signal sequence can be attached to heterologous proteins to be secreted into the media (see U.S. Patent 4,546,082). Further, the alpha-factor and its analogs have been found to secrete heterologous proteins from a variety of yeast, such as *Saccharomyces* and *Kluyveromyces* (EP 88312306.9; EP 0324274 publication, and EP 0301669). An example for use in mammalian cells is the tPA signal used for expressing Factor VIIIc light chain.

A cell has been "transformed" by a exogenous or heterologous nucleic acid when such nucleic acid as been introduced inside the cell. The transforming nucleic acid may or may not be integrated (covalently linked) into chromosomal DNA making up the genome of the cell. In prokaryotes, for example, the transforming nucleic acid may be maintained on an episomal element such as a plasmid or viral vector. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming nucleic acid.

As used herein, a "cell line" is a clone of a primary cell that is capable of stable growth *in vitro* for many generations. As used herein, nucleic acid sequences display "substantial identity" when at least about 85% (preferably at least about 90% and most preferably at least about 95%) of the nucleotides match over the defined length of the nucleotide sequences. Sequences that are substantially identical can be identified in a Southern hybridization experiment under, for example, stringent conditions as defined for that particular system. Defining appropriate hybridization conditions is within the skill of the art.

A "heterologous" region of the nucleic acid construct is an identifiable segment of a nucleic acid within a larger nucleic acid molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous coding sequence is a construct where the coding sequence itself is not found in nature (*e.g*., a cDNA where the genomic coding sequence contains introns, or synthetic sequences having codons different than the native gene).

Vectors are used to simplify manipulation of the nucleic acids which encode the chlorotoxin and chlorotoxin derivative polypeptides, either for preparation of large quantities of nucleic acids for further processing (cloning vectors) or for expression of the polypeptides (expression vectors). Vectors comprise plasmids, viruses (including phage), and integrated DNA fragments (*i.e*., fragments that are integrated into the host genome by recombination). Cloning vectors need not contain expression control sequences. However, control sequences in an expression vector include transcriptional and translational control sequences such as a transcriptional promoter, a sequence encoding suitable ribosome binding sites, and sequences which control termination of transcription and translation. The expression vector should preferably include a selection gene to facilitate the stable expression of the chlorotoxin gene and/or to identify transformed cells. However, the selection gene for maintaining expression can be supplied by a separate vector in co-transformation systems using eukaryotic host cells.

Suitable vectors generally will contain replicon (origins of replication, for use in non-integrative vectors) and control sequences which are derived from species compatible with the intended expression host. By the term "replicable" vector as used herein, it is intended to encompass vectors containing such replicons as well as vectors which are replicated by integration into the host genome. Transformed host cells are cells which have been transformed or transfected with vectors containing chlorotoxin or chlorotoxin derivative polypeptide encoding nucleic acid. The expressed polypeptides may be secreted into the culture supernatant, under the control of suitable processing signals in the expressed peptide (*e.g*. homologous or heterologous signal sequences).

Expression vectors for host cells ordinarily include an origin of replication, a promoter located upstream from the chlorotoxin or chlorotoxin derivative polypeptide coding sequence, together with a ribosome binding site, a polyadenylation site, and a transcriptional termination sequence. Those of ordinary skill will appreciate that certain of these sequences are not required for expression in certain hosts. An expression vector for use with microbes need only contain an origin of replication recognized by the host, a promoter which will function in the host, and a selection gene.

Commonly used promoters are derived from polyoma, bovine papilloma virus, CMV (cytomegalovirus, either murine or human), Rouse sarcoma virus, adenovirus, and simian virus 40 (SV40). Other control sequences (*e.g*., terminator, polyA, enhancer, or amplification sequences) can also be used.

An expression vector is constructed so that the chlorotoxin or chlorotoxin derivative polypeptide coding sequence is located in the vector with the appropriate regulatory sequences, the positioning and orientation of the coding sequence with respect to the control sequences being such that the coding sequence is transcribed and translated under the "control" of the control sequences (*i.e*., RNA polymerase which binds to the DNA molecule at the control sequences transcribes the coding sequence). The control sequences may be ligated to the coding sequence prior to insertion into a vector, such as the cloning vectors described above. Alternatively, the coding sequence can be cloned directly into an expression vector which already contains the control sequences and an appropriate restriction site. If the selected host cell is a mammalian cell, the control sequences can be heterologous or homologous to the chlorotoxin or chlorotoxin derivative polypeptide coding sequence, and the coding sequence can either be genomic DNA containing introns or cDNA.

Higher eukaryotic cell cultures may be used to express the proteins of the present invention, whether from vertebrate or invertebrate cells, including insects, and the procedures of propagation thereof are known.

Other expression vectors are those for use in eukaryotic systems. An exemplary eukaryotic expression system is that employing vaccinia virus, which is well-known in the art (see, for example, WO 86/07593). Yeast expression vectors are known in the art (see, for example, U.S. Patents 4,446,235 and 4,430,428). Another expression system is vector pHSI, which transforms Chinese hamster ovary cells (see WO 87/02062). Mammalian tissue may be cotransformed with DNA encoding a selectable marker such as dihydrofolate reductase (DHFR) or thymidine kinase and DNA encoding the chlorotoxin or chlorotoxin derivative polypeptide. If wild type DHFR gene is employed, it is preferable to select a host cell which is deficient in DHFR, thus permitting the use of the DHFR coding sequence as marker for successful transfection in hgt medium, which lacks hypoxanthine, glycine, and thymidine.

Depending on the expression system and host selected, chlorotoxin or chlorotoxin derivative polypeptide are produced by growing host cells transformed by an exogenous or heterologous DNA construct, such as an expression vector described above under conditions whereby the polypeptide is expressed. The chlorotoxin or chlorotoxin derivative polypeptide is then isolated from the host cells and purified. If the expression system secretes the protein or peptide into the growth media, the protein can be purified directly from cell-free media. The selection of the appropriate growth conditions and initial crude recovery methods are within the skill of the art.

Once a coding sequence for a chlorotoxin or chlorotoxin fragment polypeptide of the invention has been prepared or isolated, it can be cloned into any suitable vector and thereby maintained in a composition of cells which is substantially free of cells that do not contain any chlorotoxin coding sequence. As described above, numerous cloning vectors are known to those of skill in the art.

### Pharmaceutical Compositions

Pharmaceutical compositions in the present invention can be administered via parenteral, subcutaneous, intravenous, intramuscular, intraperitoneal, intrathecal, intracranial or transdermal or buccal routes. For example, an agent may be administered locally to a tumor via microinfusion. Alternatively, or concurrently, administration may be by the oral route. The dosage administered will be dependent upon the age, health, and weight of the recipient, kind of concurrent treatment, if any, frequency of treatment, and the nature of the effect desired.

While individual needs vary, determination of optimal ranges of effective amounts of each component is within the skill of the art. Dosages of chlorotoxin and/or fragments thereof of the present invention typically comprise about 1.0 ng/kg body weight to about 0.13 mg/kg body weight. In one embodiment, dosages of chlorotoxin and/or fragments thereof comprise about 1.0 ng/kg body weight to about 0.1 mg/kg body weight. In a preferred embodiment, dosages for systemic administration comprise about 0.01 µg/kg body weight to about 0.1 mg/kg body weight. In another embodiment, the dosage of chlorotoxin and/or fragments thereof comprises less than about 0.1 mg/kg body weight. More preferred dosages for systemic administration comprise about 0.1 µg/kg body weight to about 0.05 mg/kg body weight. In another preferred embodiment, the dosage of chlorotoxin and/or fragments thereof comprises less than about 0.05 mg/kg body weight. The most preferred dosages for systemic administration comprise between about 1.0 µg/kg body weight to about 0.01 mg/kg body weight. In other embodiments, the amount of chlorotoxin and/or fragments thereof administered is an amount effective to bring the concentration of chlorotoxin and/or derivatives thereof in the serum to a concentration of about 20.0, 10.0, 5.0, 2.50, 1.25, 0.625, 0.3125, 0.156, 0.078, 0.039, 0.020, 0.010,0.005, 0.003, 0.0015, 0.0008, 0.0003 or 0.0001 nM. The preferred dosages for direct administration to a site via microinfusion comprise 1 ng/kg body weight to 1 mg/kg body weight.

In addition to chlorotoxin and/or fragments thereof, the compositions of the present invention may contain suitable pharmaceutically acceptable carriers comprising excipients and auxiliaries that facilitate processing of the active compounds into preparations which can be used pharmaceutically for delivery to the site of action. Suitable formulations for parenteral administration include aqueous solutions of the active compounds in water-soluble form, for example, water-soluble salts. In addition, suspensions of the active compounds as appropriate oily injection suspensions may be administered. Suitable lipophilic solvents or vehicles include fatty oils, for example, sesame oil or synthetic fatty acid esters, for example, ethyl oleate or triglycerides. Aqueous injection suspensions may contain substances which increase the viscosity of the suspension include, for example, sodium carboxymethyl cellulose, sorbitol and dextran. Optionally, the suspension may also contain stabilizers. Liposomes can also be used to encapsulate the agent for delivery into the cell.

The pharmaceutical formulation for systemic administration according to the invention may be formulated for enteral, parenteral or topical administration. Indeed, all three types of formulations may be used simultaneously to achieve systemic administration of the active ingredient.

As mentioned above, topical administration may be used. Any common topical formulation such as a solution, suspension, gel, ointment or salve and the like may be employed. Preparation of such topical formulations are described in the art of pharmaceutical formulations as exemplified, for example, by Gennaro et al. (1995) Remington's Pharmaceutical Sciences, Mack Publishing. For topical application, the compositions could also be administered as a powder or spray, particularly in aerosol form. In a some embodiments, the compositions of this invention may be administered by inhalation. For inhalation therapy the active ingredients may be in a solution useful for administration by metered dose inhalers or in a form suitable for a dry powder inhaler. In another embodiment, the compositions are suitable for administration by bronchial lavage.

Suitable formulations for oral administration include hard or soft gelatin capsules, pills, tablets, including coated tablets, elixirs, suspensions, syrups or inhalations and controlled release forms thereof.

As used herein, the term "chlorotoxin or a fragment thereof" also includes pharmaceutically acceptable salts, solvates, hydrates, clathrates, polymorphs and prodrugs thereof. According to the invention, the chemical structures depicted herein, and therefore the polypeptides useful in the invention, encompass all of the corresponding enantiomers and stereoisomers, that is, both the stereomerically pure form (*e.g*., geometrically pure, enantiomerically pure, or diastereomerically pure) and enantiomeric and stereoisomeric mixtures. Enantiomeric and stereoisomeric mixtures can be resolved into their component enantiomers or stereoisomers by well known methods, such as chiral-phase gas chromatography, chiral-phase high performance liquid chromatography, crystallizing the compound as a chiral salt complex, or crystallizing the compound in a chiral solvent.

Enantiomers and stereoisomers can also be obtained from stereomerically- or enantiomerically-pure intermediates, reagents, and catalysts by well known asymmetric synthetic methods.

The invention includes chlorotoxin, fragments thereof, or pharmaceutically acceptable salts, solvates, hydrates, clathrates, polymorphs and prodrugs thereof and a pharmaceutically acceptable vehicle.

As used herein and unless otherwise indicated, the term "pharmaceutically acceptable" means approved by a regulatory agency of the Federal or a state government or listed in the U.S. Pharmacopeia or other generally recognized pharmacopeia for use in animals, and more particularly in humans. The term "vehicle" refers to a diluent, adjuvant, excipient, or carrier with which a compound of the invention is administered. Such pharmaceutical vehicles can be, for example, liquids, such as water and oils, including those of petroleum, animal, vegetable or synthetic origin, such as peanut oil, soybean oil, mineral oil, sesame oil and the like. The pharmaceutical vehicles can be saline, methyl cellulose, gum acacia, gelatin, starch paste, talc, keratin, colloidal silica, urea, and the like. In addition, auxiliary, stabilizing, thickening, lubricating and coloring agents may be used. When administered to a patient, the compositions of the invention and pharmaceutically acceptable vehicles are preferably sterile. Water is a preferred vehicle when the composition of the invention is administered intravenously. Saline solutions and aqueous dextrose and glycerol solutions can also be employed as liquid vehicles, particularly for injectable solutions. Suitable pharmaceutical vehicles also include excipients such as starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The present compositions, if desired, can also contain minor amounts of wetting or emulsifying agents, or pH buffering agents.

A.s used herein and unless otherwise indicated, the phrase "pharmaceutically acceptable salt" includes, but is not limited to, salts of acidic or basic groups that may be present in compositions. Polypeptides included in the present compositions that are basic in nature are capable of forming a wide variety of salts with various inorganic and organic acids. The acids that may be used to prepare pharmaceutically acceptable acid addition salts of such basic compounds are those that form non-toxic acid addition salts, (*i.e.,* salts containing pharmacologically acceptable anions), including, but not limited to, sulfuric, citric, maleic, acetic, oxalic, hydrochloride, hydrobromide, hydroiodide, nitrate, sulfate, bisulfate, phosphate, acid phosphate, isonicotinate, acetate, lactate, salicylate, citrate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate (*i.e*., 1,1'-methylene-bis-(2-hydroxy-3-naphthoate) salts. Polypeptides included in compositions used in the methods of the invention that are acidic in nature are capable of forming base salts with various pharmacologically acceptable cations. Examples of such salts include alkali metal or alkaline earth metal salts and, particularly, calcium, magnesium, sodium lithium, zinc, potassium, and iron salts.

As used herein and unless otherwise indicated, the term "pharmaceutically acceptable solvate" means a chlorotoxin polypeptide or derivative thereof that further includes a stoichiometric or non-stoichiometric amount of a solvent bound by non-covalent intermolecular forces. Preferred solvents are volatile, non-toxic, and/or acceptable for administration to humans in trace amounts.

As used herein and unless otherwise indicated, the term "pharmaceutically acceptable hydrate" means a chlorotoxin polypeptide or derivative thereof that further includes a stoichiometric or non-stoichiometric amount of water bound by non-covalent intermolecular forces.

As used herein and unless otherwise indicated, the term "pharmaceutically acceptable clathrate" means a chlorotoxin polypeptide or derivative thereof of the invention that is in the form of a crystal lattice that contains spaces (*e.g*., channels) that have a guest molecule (*e.g*., a solvent or water) trapped within.

As used herein and unless otherwise indicated, the term "pharmaceutically acceptable polymorph" refers to a chlorotoxin polypeptide or derivative thereof that exists in several distinct forms (*e.g*., crystalline, amorphous), the invention encompasses all of these forms. Polymorphs are, by definition, crystals of the same molecule having different physical properties as a result of the order of the molecules in the crystal lattice. The differences in physical properties exhibited by polymorphs affect pharmaceutical parameters such as storage stability, compressibility and density (important in formulation and product manufacturing), and dissolution rates (an important factor in determining bio-availability). Differences in stability can result from changes in chemical reactivity (*e.g*., differential oxidation, such that a dosage form discolors more rapidly when comprised of one polymorph than when comprised of another polymorph) or mechanical changes (*e.g*., tablets crumble on storage as a kinetically favored polymorph converts to thermodynamically more stable polymorph) or both (*e.g*., tablets of one polymorph are more susceptible to breakdown at high humidity). As a result of solubility/dissolution differences, in the extreme case, some polymorphic transitions may result in lack of potency or, at the other extreme, toxicity. In addition, the physical properties of the crystal may be important in processing: for example, one polymorph might be more likely to form solvates or might be difficult to filter and wash free of impurities (*i.e*., particle shape and size distribution might be different between one polymorph relative to the other).

As used herein and unless otherwise indicated, the term "pharmaceutically acceptable prodrug" means a derivative of a compound that can hydrolyze, oxidize, or otherwise react under biological conditions (*in vitro* or *in vivo*) to provide the compound. Examples of prodrugs include, but are not limited to, compounds that comprise biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include compounds that comprise oligonucleotides, peptides, lipids, aliphatic and aromatic groups, or NO, ONO, NO₂ and ONO₂ moieties. Prodrugs can typically be prepared using well known methods, such as those described in Bungaard (1985) Design of Prodrugs, Elselvier Press.

As used herein and unless otherwise indicated, the terms "biohydrolyzable amide" or "biohydrolyzable ester" or "biohydrolyzable carbamate" or "biohydrolyzable carbonate" or "biohydrolyzable ureide" or "biohydrolyzable phosphate" mean an amide, ester, carbamate, carbonate, ureide, or phosphate, respectively, of a compound that either: (1) does not interfere with the biological activity of the compound but can confer upon that compound advantageous properties *in vivo*, such as uptake, duration of action, or onset of action; or (2) is biologically inactive but is converted in vivo to the biologically active compound. Examples of biohydrolyzable esters include, but are not limited to, lower alkyl esters, lower acyloxyalkyl esters (such as acetoxylmethyl, acetoxyethyl, aminocarbonyloxy-methyl, pivaloyloxymethyl, and pivaloyloxyethyl esters), lactonyl esters (such as phthalidyl and thiophthalidyl esters), lower alkoxyacyloxyalkyl esters (such as methoxycarbonyloxy-methyl, ethoxycarbonyloxyethyl and isopropoxycarbonyloxyethyl esters), alkoxyalkyl esters, choline esters, and acylamino alkyl esters (such as acetamidomethyl esters). Examples of biohydrolyzable amides include, but are not limited to, lower alkyl amides, a amino acid amides, alkoxyacyl amides, and alkylaminoalkyl-carbonyl amides. Examples of biohydrolyzable carbamates include, but are not limited to, lower alkylamines, substituted ethylenediamines, aminoacids, hydroxyalkylamines, heterocyclic and heteroaromatic amines, and polyether amines.

As used herein and unless otherwise indicated, the term "therapeutically effective" refers to an amount of a chlorotoxin polypeptide or derivative thereof or a pharmaceutically acceptable salt, solvate, hydrate, clathrate, polymorph, or prodrug thereof able to cause an amelioration of a disease or disorder, or at least one discernible symptom thereof. "Therapeutically effective" also refers to an amount that results in an amelioration of at least one measurable physical parameter, not necessarily discernible by the patient In yet another embodiment, the term "therapeutically effective" refers to an amount that inhibits the progression of a disease or disorder, either physically (*e.g*., stabilization of a discernible symptom), physiologically (*e.g*., stabilization of a physical parameter), or both. In yet another embodiment, the term "therapeutically effective" refers to an amount that results in a delayed onset of a disease or disorder.

As used herein and unless otherwise indicated, the term "prophylactically effective" refers to an amount of a chlorotoxin polypeptide or derivative thereof, or a pharmaceutically acceptable salt, solvate, hydrate, clathrate, polymorph, or prodrug thereof causing a reduction of the risk of acquiring a given disease or disorder. In one embodiment, the compositions are administered as a preventative measure to an animal, preferably a human, having a genetic predisposition to a disorder described herein. In another embodiment of the invention, the compositions are administered as a preventative measure to a patient having a non-genetic predisposition to a disorder disclosed herein. Accordingly, the compositions of the invention may be used for the prevention of one disease or disorder and concurrently treating another.

The invention also includes the use of isotopically-labeled chlorotoxin and chlorotoxin fragments that have one or more atoms replaced by an atom having an atomic mass or mass number different from the atomic mass or mass number usually found in nature, or one or more of such atoms attached to the chlorotoxin derivatives. Examples of isotopes that can be incorporated include, but are not limited to, isotopes of hydrogen, carbon, phosphorous, iodine, rhenium, indium, yttrium, technetium and lutetium (*i.e*., including, but not limited to, ³H, ¹⁴C, ³¹P, ³¹P, ³⁵S, ¹³¹I, ¹²⁵I, ¹²³I ¹⁸⁷Re, ⁶⁴Cu, ¹¹¹In, ⁹⁰Y, ^{99m}Tc, ¹⁷⁷Lu), others isotopes of these elements, and other isotopes known in the art. Labeled chlorotoxin, chlorotoxin fragments, prodrugs thereof, and pharmaceutically acceptable salts which contain the aforementioned isotopes and/or other isotopes of other atoms can be used for the diagnosis and treatment of myleoproliferative or lymphoproliferative diseases. Tritium and carbon-14 isotopes are particularly preferred for their ease of preparation and detectability. Further, substitution with heavier isotopes such as deuterium can afford certain therapeutic advantages resulting from greater metabolic stability, for example increased *in vivo* half-life or reduced dosage requirements and, hence, may be preferred in some circumstances.

### Fusion Proteins

Where the present invention relates to treatment, a cytotoxic agent is linked to chlorotoxin. Examples of cytotoxic agents include, but are not limited to, gelonin, ricin, saponin, pseudonomas exotoxin, pokeweed antiviral protein, diphtheria toxin, complement proteins, or any other agent known in the art which is capable of killing a cell upon contact with that cell.

Where the present invention relates to diagnosis, chlorotoxin may be linked to at least one second polypeptide. In some embodiments, the second polypeptide includes a binding domain which specifically binds to an epitope expressed only on myeloid and/or lymphoid cells exhibiting abnormal growth (*i.e*. cancer cells). These binding domains have an amino acid sequence capable of binding or otherwise specifically associating with a myeloid and/or lymphoid cell displaying abnormal growth (*e.g.,* benign and malignant cancer cells). In some embodiments the binding-domain is an antibody while in other embodiments it is a ligand which specifically binds to a receptor expressed only on these cells. Examples of antibodies include, but are not limited to, antibodies which specifically bind to B-cells or T-cells. Examples of receptor ligands include, but are not limited to, cytokines and growth factors including epidermal growth factor.

The second polypeptide can also include a stabilization domain which increases the *in vitro* and *in vivo* half-life of the fusion polypeptide. As used herein, the term "stabilization domain" refers to an amino acid sequence capable of extending the *in vitro* and *in vivo* half-life of chlorotoxin or a chlorotoxin derivative when compared to chlorotoxin alone. The stabilization domain can comprise human proteins (*e.g*., full length or truncated, soluble proteins from extracellular fragments, etc.) such as human serum albumin or other proteins which stabilize the *in vivo* or *in vitro* half-life of chlorotoxin or a chlorotoxin derivative. These additional functional domains may themselves serve as linker peptides, for example, for joining a cancer cell-binding domain to chlorotoxin or a chlorotoxin derivative. Alternatively, they may be located elsewhere in the fusion molecule (*e.g*., at the amino or carboxy terminus thereof). In alternative embodiments, the stabilization domain is a chemical moiety (*e.g*., PEG (polyethylene glycol) or a dextran).

The term "fused" or "fusion polypeptide" as used herein refers to polypeptides in which: (i) a given functional domain (*i.e*. a cancer cell-binding domain) is bound at its carboxy terminus by a covalent bond either to the amino terminus of another functional domain (*i.e*., an human serum albumin component) or to a linker peptide which itself is bound by a covalent bond to the amino terminus of chlorotoxin or a chlorotoxin derivative; or (ii) a given functional domain (*i.e*. a chlorotoxin binding domain) is bound at its amino terminus by a covalent bond either to the carboxy terminus of another functional domain (*i.e.,* an human serum albumin component) or to a linker peptide which itself is bound by a covalent bond to the carboxy terminus of chlorotoxin or a chlorotoxin derivative.

Similarly, "fused" when used in connection with nucleic acid intermediates means that the 3'- [or 5'-] terminus of a nucleotide sequence encoding a first functional domain is bound to the respective 3'- [or 5'-] terminus of a nucleotide sequence encoding a second functional domain, either by a covalent bond or indirectly via a peptide linker which itself is covalently bound preferably at its termini to the first functional domain-encoding polynucleotide and optionally, a second functional domain-encoding nucleic acid.

Examples of fusion polypeptides may be represented by, but are not limted by, the following formulas:

R1-L-R2 (i)

R2-L-R1 (ii)

R1-L-R2-L-R1 (iii)

R1-L-R1-L-R2 (iv)

R2-L-R1-L-R1 (iv)

wherein R1 is the amino acid sequence of a domain which binds specifically to a myeloid and/or lymphoid cancer cell or is a transport domain, for instance a domain that allows entry of the polypeptide across the blood-brain barrier, R2 is the amino acid sequence of a stabilizing domain (*e.g*., human serum albumin), each L is chlorotoxin or a chlorotoxin derivative which is bound by a covalent bond to a terminus of R1 and/or R2, whereby the above molecule fragments are read directionally (*i.e.,* with the left side corresponding to the amino terminus and the right side to the carboxy terminus of the molecule).

### Diagnosis Using Chlorotoxin and/or Fragments Thereof

In practicing diagnosis in accordance with this invention, chlorotoxin and/or derivatives thereof may be used alone or in combination with other inactive ingredients. As discussed above, the present invention includes compositions and methods where a label is linked to chlorotoxin or a derivative thereof. The invention therefore includes administration of labeled chlorotoxin or a labeled derivative thereof for the diagnosis of a disease associated with abnormal myeloid or lymphoid cell growth, including cancer.

The invention can be utilized both *in vivo*, ordinarily in mammals, such as humans, sheep, horses, cattle, pigs, dogs, cats, rats and mice or *in vitro.* The invention is particularly useful in the diagnosis of human subjects.

Without further description, it is believed that one of ordinary skill in the art can, using the preceding description and the following illustrative examples, make and utilize the compounds of the present invention and practice the claimed methods. The following working examples describe embodiments of the present invention, and are not to be construed as limiting in any way the remainder of the disclosure.

### Example 1

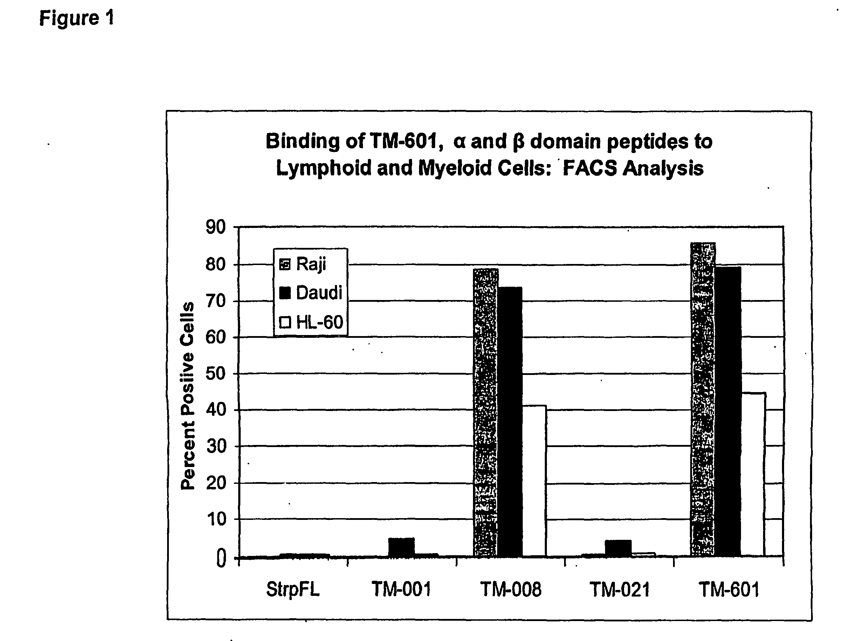

Raji (Epstein et al. (1966) J. Natl. Cancer Inst. 37, 547-559) and Daudi (Klein et al. (1968) Cancer Res. 28,1300-1310) cells were used either unfixed or fixed with one percent glutaraldehyde. The Raji cell line (ATCC CCL-86) is a B lymphocyte cell line derived from a Burkitt's lymphoma of an eleven year old black male. The Daudi cell line (ATCC CCL-213) is a B lymphoblast cell line derived from sixteen year old black male. Cells were incubated with chlorotoxin or the designated binding domain peptide (SEQ ID NO: 8 or 10), washed then incubated with streptavidin-fluorescein (strp-FL). After another wash, cells were analyzed using a FACS analyzer and the percent of positive staining cells determined (Figure 1). Controls included untreated cells, cells incubated with streptavidin-fluorescein alone and cells incubated with negative peptide.

### Example 2

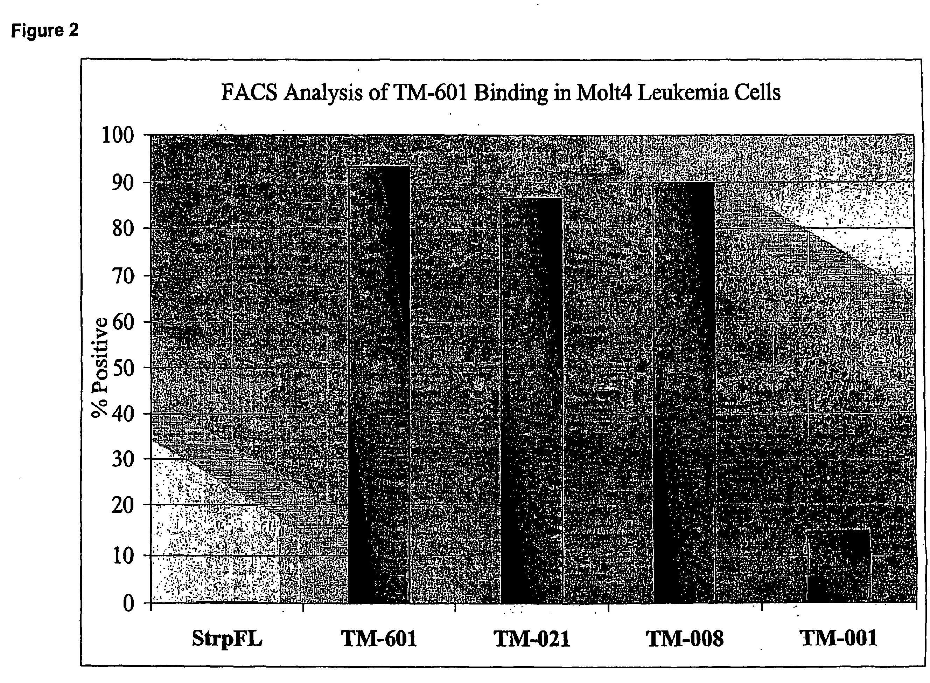

Molt-4 cells (Minowada et al. (1972) J. Natl. Cancer Inst. 49, 891-895) were used either unfixed or fixed with one percent glutaraldehyde. The Molt-4 cell line is a T lymphoblast cell line derived from a patient in relapse. Cells were incubated with chlorotoxin or the designated binding domain peptide (SEQ ID NO: 8 or 10), washed then incubated with streptavidin-fluorescein (strp-FL). After another wash, cells were analyzed using a FACS analyzer and the percent of positive staining cells determined (Figure 2). Controls included untreated cells, cells incubated with streptavidin-fluorescein alone and cells incubated with negative peptide.

### Example 3

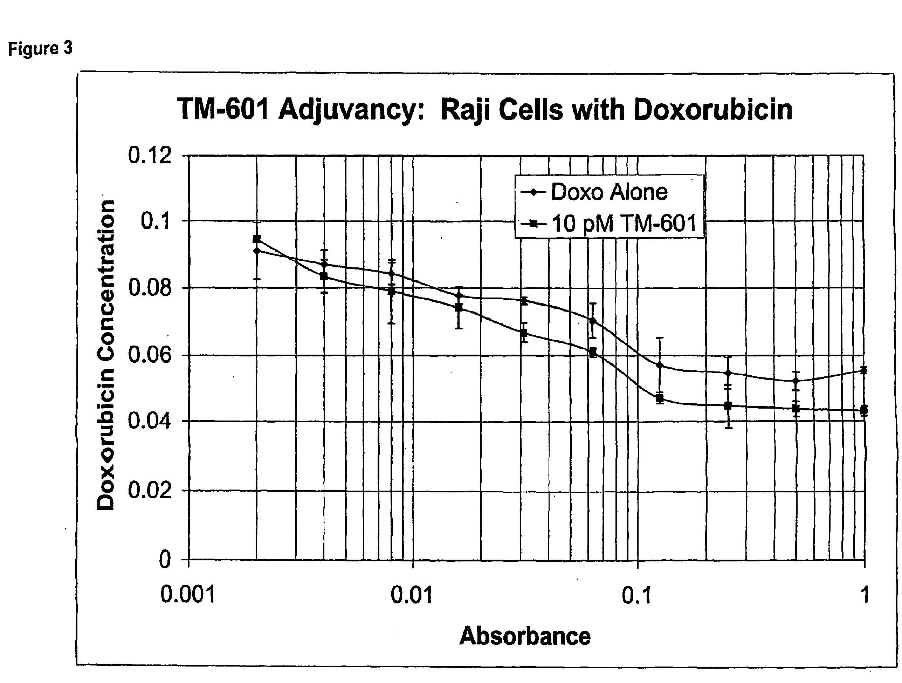

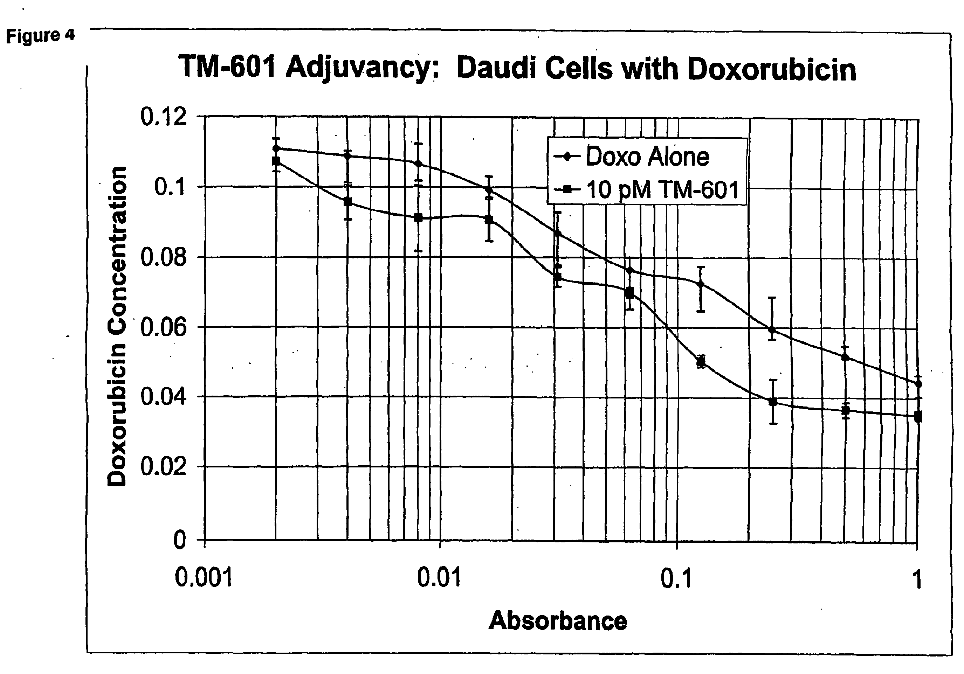

A tissue culture method was optimized to test the effects of chlorotoxin (TM-601) in the presence or absence of doxorubicin on Raji (Figure 3) and Daudi (Figure 4) cell lines. Cells were plated on 96-well microtiter tissue culture plates at a density of approximately 1000-2000 cells per well, depending on the specific cell line. Cells were allowed to adhere twenty-four hours in a 37°C, humidified cell culture incubator supplied with five percent carbon dioxide. In order to achieve a dose-response curve for each drug in each cell line, cells were treated with decreasing concentrations doxorubicin for two to five days. Following treatment, the cytotoxic effect of doxorubicin was quantified using the Cell Counting Kit-8 (CCK-8) (Dojindo Inc.) according to the manufacturer's instructions. In brief, following the treatment period with doxorubicin, cells were incubated with CCK-8 reagent and incubated at 37°C for one to four hours, depending on the specific cell type. After incubation, plates were read on a microplate reader at a wavelength of 490 nm.

## Claims

1. A composition comprising chlorotoxin (SEQ ID NO: 1) or a fragment thereof having the amino acid sequence KGRGKSY (SEQ ID NO: 8) for use in the diagnosis of a lymphoproliferative or myeloproliferative disorder in a mammal, wherein the composition is administered to the mammal and detection of binding of the composition in the mammal indicates the presence of a lymphoproliferative or myeloproliferative disorder in the mammal.

2. The composition of claim 1, wherein the chlorotoxin or fragment thereof is linked to a second polypeptide.

3. The composition of claim 2, wherein the second polypeptide is an antibody or fragment thereof which binds specifically to an epitope expressed only by a myeloid or lymphoid cancer cell.

4. The composition of claim 2, wherein the second polypeptide is selected from the group consisting of polyhistidine and human serum albumin.

5. A composition comprising chlorotoxin (SEQ ID NO: 1) or a fragment thereof having the amino acid sequence KGRGKSY (SEQ ID NO: 8) for use in treating a lymphoproliferative or myeloproliferative disorder in a mammal, wherein the chlorotoxin or fragment thereof is linked to a cytotoxic agent.

6. The composition of claim 5, wherein the cytotoxic agent is selected from the group consisting of gelonin, ricin, saponin, pseudonomas exotoxin, pokeweed antiviral protein, diphtheria toxin and complement proteins.

7. The composition of claim 5 or claim 6, wherein the mammal is a human and wherein the amount of chlorotoxin administerable comprises between 0.01 µg/kg body weight to 2.0 mg/kg body weight.

8. The composition of any preceding claim, wherein the lymphoproliferative disorders is a non-Hodgkin's lymphoma.

9. The composition of claim 8, wherein the non-Hodgkin's lymphoma is a B cell neoplasm or a T cell neoplasm.

10. The composition of claim 9, wherein the B cell neoplasm is a Precursor B cell lymphoblastic leukaemia/lymphoma or a mature B cell neoplasm.

11. The composition of claim 10, wherein the mature B cell neoplasm is selected from the group consisting of B cell chronic lymphocytic leukaemia/small lymphocytic lymphoma, B cell prolymphocytic leukaemia, Lymphoplasmacytic lymphoma, Splenic marginal zone B cell lymphoma, Hairy cell leukaemia, Extranodal marginal zone B cell lymphoma, Mantle cell lymphoma, Follicular lymphoma, Nodal marginal zone lymphoma, Diffuse large B cell lymphoma, Burkitt's lymphoma, Plasmacytoma, and Plasma cell myeloma.

12. The composition of claim 9, wherein the T cell neoplasm is selected from the group consisting of T cell prolymphocytic leukaemia, T cell large granular lymphocytic leukaemia, NK cell leukaemia, Extranodal NK/T cell lymphoma, Mycosis fungoides, Primary cutaneous anaplastic large cell lymphoma, Subcutaneous panniculitis-like T cell lymphoma, Enteropathy-type intestinal T cell lymphoma, Hepatosplenic gamma-delta T cell lymphoma, Angioimmunoblastic T cell lymphoma, Peripheral T cell lymphoma, Anaplastic large cell lymphoma and Adult T cell lymphoma.

13. The composition of any preceding claim, wherein the mammal is a human.

14. The composition of any preceding claim , wherein the myeloproliferative disorder is selected from the group consisting of polycythemia vera (PV), essential thrombocythemia (ET), agnogenic myeloid metaplasia (AMM) also referred to as idiopathic myelofibrosis (IMF) and chronic myelogenous leukaemia (CML).

15. The composition of claim 1, wherein the chlorotoxin or fragment thereof is labelled.

16. The composition of claim 15, wherein the label is ¹³¹I.

17. A method of detecting the presence of a lymphoproliferative or a myeloproliferative disorder in a mammal comprising:
(a) contacting a biological sample isolated from the mammal with a composition comprising chlorotoxin (SEQ ID NO: 1) or a fragment thereof having the amino acid sequence KGRGKSY (SEQ ID NO: 8), and
(b) detecting binding of the composition to the sample, wherein the binding indicates presence of a lymphoproliferative or myeloproliferative disorder in the mammal.

18. The method of claim 17, modified by the features of any one of claims 2 to 4, and 8 to 16.

19. The use of a composition comprising chlorotoxin (SEQ ID NO:1) or a fragment thereof having the amino acid sequence KGRGKSY (SEQ ID NO:8) in the manufacture of a medicament for treating a lymphoproliferative or myeloproliferative disorder in a mammal, wherein the chlorotoxin or fragment thereof is linked to a cytotoxic agent.

20. The use of claim 19, modified by the features of any one of claims 6 to 14.

## Patentansprüche

1. Zusammensetzung, umfassend Chlorotoxin (SEQ ID Nr. 1) oder ein Fragment davon mit der Aminosäuresequenz KGRGKSY (SEQ ID Nr. 8) zur Verwendung in der Diagnose einer lymphoproliferativen oder myeloproliferativen Störung in einem Säuger, wobei die Zusammensetzung an den Säuger verabreicht wird und Nachweis von Bindung der Zusammensetzung in dem Säuger die Anwesenheit einer lymphoproliferativen oder myeloproliferativen Störung in dem Säuger anzeigt.

2. Zusammensetzung nach Anspruch 1, wobei das Chlorotoxin oder Fragment davon an ein zweites Polypeptid gebunden ist.

3. Zusammensetzung nach Anspruch 2, wobei das zweite Polypeptid ein Antikörper oder Fragment davon ist, welches spezifisch an ein Epitop bindet, welches nur durch eine myeoloide oder lymphoide Krebszelle exprimiert wird.

4. Zusammensetzung nach Anspruch 2, wobei das zweite Polypeptid ausgewählt ist aus der Gruppe bestehend aus Polyhistidin und menschlichem Serumalbumin.

5. Zusammensetzung, umfassend Chlorotoxin (SEQ ID Nr. 1) oder ein Fragment davon mit der Aminosäuresequenz KGRGKSY (SEQ ID Nr. 8) zur Verwendung beim Behandeln einer lymphoproliferativen oder myeloproliferativen Störung in einem Säuger, wobei das Chlorotoxin oder Fragment davon an einen zytotoxischen Wirkstoff gebunden ist.

6. Zusammensetzung nach Anspruch 5, wobei der zytotoxische Wirkstoff ausgewählt ist aus der Gruppe bestehend aus Gelonin, Ricin, Saponin, Pseudonomas-Exotoxin, Pokeweed-Antiviral-Protein, Diphtheria-Toxin und Komplementproteinen.

7. Zusammensetzung nach Anspruch 5 oder Anspruch 6, wobei der Säuger ein Mensch ist und wobei die Menge an verabreichbarem Chlorotoxin zwischen 0,01 µg/kg Körpergewicht bis 2,0 mg/kg Körpergewicht umfasst.

8. Zusammensetzung nach einem vorhergehenden Anspruch, wobei die lymphoproliferative Störung ein Non-Hodgkin-Lymphom ist.

9. Zusammensetzung nach Anspruch 8, wobei das Non-Hodgkin-Lymphom ein B-Zell-Neoplasma oder ein T-Zell-Neoplasma ist.

10. Zusammensetzung nach Anspruch 9, wobei das B-Zell-Neoplasma ein Vorläufer-B-Zell-lymphoblastisches Leukämie/Lymphom- oder ein reifes B-Zell-Neoplasma ist.

11. Zusammensetzung nach Anspruch 10, wobei das reife B-Zell-Neoplasma ausgewählt ist aus der Gruppe bestehend aus B-Zell chronischem lymphozytischen Leukämie/kleinzelligen lymphozytischen Lymphom, B-Zell prolymphozytischer Leukämie, lymphoplasmazytischem Lymphom, Marginalzonen-B-Zell-Lymphom der Milz, Haar-Zell-Leukämie, extranodalem Marginalzonen-B-Zell-Lymphom, Mantelzelllymphom, follikulärem Lymphom, nodalem Marginalzonenlymphom, diffusem großzelligen B-Zell-Lymphom, Burkitt-Lymphom, Plasmazytom und Plasmazellmyelom.

12. Zusammensetzung nach Anspruch 9, wobei das T-Zell-Neoplasma ausgewählt ist aus der Gruppe bestehend aus T-Zell prolymphozytischer Leukämie, T-Zell großer granulärer lymphozytischer Leukämie, NK-Zell-Leukämie, extranodalem NK/T-Zell-Lymphom, Mycosis fungoides, primärem kutanen anaplastischen großzelligen Lymphom, subkutanem Pannikulitis-ähnlichem T-Zell-Lymphom, intestinalem T-Zell-Lymphom vom Enteropathie-Typ, hepatosplenalem Gamma-Delta-T-Zell-Lymphom, angioimmunoblastischem T-Zell-Lymphom, peripherem T-Zell-Lymphom, anaplastischem großzelligen Lymphom und adultem T-Zell-Lymphom.

13. Zusammensetzung nach einem vorhergehenden Anspruch, wobei der Säuger ein Mensch ist.

14. Zusammensetzung nach einem vorhergehenden Anspruch, wobei die myeloproliferative Störung ausgewählt ist aus der Gruppe bestehend aus Polycythaemia vera (PV), essentieller Trombozythämie (ET), agnogener myeloischer Metaplasie (AMM), auch bezeichnet als idiopathische Myelofibrose (IMF) und chronischer myelogener Leukämie (CML).

15. Zusammensetzung nach Anspruch 1, wobei das Chlorotoxin oder Fragment davon markiert ist.

16. Zusammensetzung nach Anspruch 15, wobei die Markierung ¹³¹I ist.

17. Verfahren des Nachweisens der Anwesenheit einer lymphoproliferativen oder einer myeloproliferativen Störung in einem Säuger, welches umfasst:
(a) Kontaktieren einer biologischen Probe, isoliert aus dem Säuger, mit einer Zusammensetzung, umfassend Chlorotoxin (SEQ ID Nr. 1) oder ein Fragment davon mit der Aminosäuresequenz KGRGKSY (SEQ ID Nr. 8), und
(b) Nachweisen von Bindung der Zusammensetzung an die Probe, wobei die Bindung Anwesenheit einer lymphoproliferativen oder myeloproliferativen Störung in dem Säuger anzeigt.

18. Verfahren nach Anspruch 17, modifiziert durch die Kennzeichen nach einem von Ansprüchen 2 bis 4 und 8 bis 16.

19. Verwendung einer Zusammensetzung, umfassend Chlorotoxin (SEQ ID Nr. 1) oder ein Fragment davon mit der Aminosäuresequenz KGRGKSY (SEQ ID Nr. 8) in der Herstellung eines Medikaments zum Behandeln einer lymphoproliferativen oder myeloproliferativen Störung in einem Säuger, wobei das Chlorotoxin oder Fragment davon an einen zytotoxischen Wirkstoff gebunden ist.

20. Verwendung nach Anspruch 19, modifiziert durch die Kennzeichen nach einem von Ansprüchen 6 bis 14.

## Revendications

1. Composition comprenant de la chlorotoxine (SEQ ID NO : 1) ou un fragment de celle-ci ayant la séquence d'acides aminés KGRGKSY (SEQ ID NO : 8) en vue d'une utilisation dans le diagnostic d'un trouble lymphoprolifératif ou myéloprolifératif chez un mammifère, dans laquelle la composition est administrée au mammifère et la détection de la liaison de la composition chez le mammifère indique la présence d'un trouble lymphoprolifératif ou myéloprolifératif chez le mammifère.

2. Composition selon la revendication 1, dans laquelle la chlorotoxine ou un fragment de celle-ci est lié à un second polypeptide.

3. Composition selon la revendication 2, dans laquelle le second polypeptide est un anticorps ou un fragment de celui-ci qui se lie spécifiquement à un épitope exprimé uniquement par une cellule cancéreuse myéloïde ou lymphoïde.

4. Composition selon la revendication 2, dans laquelle le second polypeptide est choisi dans le groupe constitué de polyhistidine et de sérumalbumine humaine.

5. Composition comprenant de la chlorotoxine (SEQ ID NO : 1) ou un fragment de celle-ci ayant la séquence d'acides aminés KGRGKSY (SEQ ID NO : 8) en vue d'une utilisation dans le traitement d'un trouble lymphoprolifératif ou myéloprolifératif chez un mammifère, dans laquelle la chlorotoxine ou un fragment de celle-ci est lié à un agent cytotoxique.

6. Composition selon la revendication 5, dans laquelle l'agent cytotoxique est choisi dans le groupe constitué de gélonine, ricine, saponine, exotoxine de Pseudomonas, protéine antivirale de la phytolaque, toxine diphtérique et protéines du complément.

7. Composition selon la revendication 5 ou la revendication 6, dans laquelle le mammifère est un être humain et dans laquelle la quantité de chlorotoxine administrable est comprise entre 0,01 µg/kg de poids corporel et 2,0 mg/kg de poids corporel.

8. Composition selon l'une quelconque des revendications précédentes, dans laquelle le trouble lymphoprolifératif est un lymphome non hodgkinien.

9. Composition selon la revendication 8, dans laquelle le lymphome non hodgkinien est un néoplasme à cellules B ou un néoplasme à cellules T.

10. Composition selon la revendication 9, dans laquelle le néoplasme à cellules B est une leucémie/un lymphome lymphoblastique à précurseurs de cellules B ou un néoplasme à cellules B matures.

11. Composition selon la revendication 10, dans laquelle le néoplasme à cellules B matures est choisi dans le groupe constitué de leucémie à lymphocytes B/lymphome chronique à petits lymphocytes B, leucémie prolymphocytaire à cellules B, lymphome lymphoplasmacytaire, lymphome à cellules B de la zone marginale splénique, leucémie à trichocytes, lymphome à cellules B de la zone marginale extra-ganglionnaire, lymphome centrocytique, lymphome folliculaire, lymphome de la zone marginale ganglionnaire, lymphome diffus à grandes cellules B, lymphome de Burkitt, plasmacytome et myélome à cellules plasmatiques.

12. Composition selon la revendication 9, dans laquelle le néoplasme à cellules T est choisi dans le groupe constitué de leucémie prolymphocytaire à cellules T, leucémie à grands lymphocytes T granuleux, leucémie à cellules NK, lymphome à cellules NK/T extra-ganglionnaire, mycosis fongoïdes, lymphome cutané primaire à grandes cellules anaplasiques, lymphome sous-cutané à cellules T de type panniculite, lymphome intestinal à cellules T de type entéropathie, lymphome gamma-delta hépatosplénique à cellules T, lymphome angio-immunoblastique à cellules T, lymphome périphérique à cellules T, lymphome à grandes cellules anaplasiques et lymphome de l'adulte à cellules T.

13. Composition selon l'une quelconque des revendications précédentes, dans laquelle le mammifère est un être humain.

14. Composition selon l'une quelconque des revendications précédentes, dans laquelle le trouble myéloprolifératif est choisi dans le groupe constitué de polyglobulie essentielle (PE), thrombocytémie essentielle (TE), métaplasie myéloïde agnogénique (MMA) également appelée myélofibrose idiopathique (MFI) et leucémie myélogène chronique (LMC).

15. Composition selon la revendication 1, dans laquelle la chlorotoxine ou un fragment de celle-ci est marqué.

16. Composition selon la revendication 15, dans laquelle le marqueur est ¹³¹I.

17. Procédé de détection de la présence d'un trouble lymphoprolifératif ou myéloprolifératif chez un mammifère comprenant :
(a) la mise en contact d'un échantillon biologique isolé du mammifère avec une composition comprenant de la chlorotoxine (SEQ ID NO : 1) ou un fragment de celle-ci ayant la séquence d'acides aminés KGRGKSY (SEQ ID NO : 8), et
(b) la détection de la liaison de la composition à l'échantillon, dans lequel la liaison indique la présence d'un trouble lymphoprolifératif ou myéloprolifératif chez le mammifère.

18. Procédé selon la revendication 17, modifié par les caractères selon l'une quelconque des revendications 2 à 4 et 8 à 16.

19. Utilisation d'une composition comprenant de la chlorotoxine (SEQ ID NO : 1) ou un fragment de celle-ci ayant la séquence d'acides aminés KGRGKSY (SEQ ID NO : 8) dans la fabrication d'un médicament destiné au traitement d'un trouble lymphoprolifératif ou myéloprolifératif chez un mammifère, dans laquelle la chlorotoxine ou un fragment de celle-ci est lié à un agent cytotoxique.

20. Utilisation selon la revendication 19, modifiée par les caractères selon l'une quelconque des revendications 6 à 14.


### SEQUENCE LISTING

<110> Vernon, et al.
<120> Diagnosis and Treatment of Myeloid and Lymphoid cell Cancers
<130> 2006636-0079
<140> PCT/US2005/011523
   <141> 2005-04-06
<150> 60/559,433
   <151> 2004-04-06
<160> 34
<170> PatentIn version 3.2
<210> 1
   <211> 36
   <212> PRT
   <213> Leiurus quinquestriatus
<220>
   <221> MISC_FEATURE
   <223> Chlorotoxin
<400> 1
   Met Cys Met Pro Cys Phe Thr Thr Asp His Gln Met Ala Arg Lys Cys
   1               5                   10                  15
   Asp Asp Cys Cys Gly Gly Lys Gly Arg Gly Lys Cys Tyr Gly Pro Gln
               20                  25                  30
   Cys Leu Cys Arg
           35
<210> 2
   <211> 42
   <212> PRT
   <213> Leiurus quinquestriatus
<400> 2
   His His His His His His Met Cys Met Pro Cys Phe Thr Thr Asp His
   1               5                   10                  15
   Gln Met Ala Arg Lys Cys Asp Asp Cys Cys Gly Gly Lys Gly Arg Gly
               20                  25                  30
   Lys Cys Tyr Gly Pro Gln Cys Leu Cys Arg
           35                  40
<210> 3
   <211> 37
   <212> PRT
   <213> Leiurus quinquestriatus
<400> 3
   Tyr Met Cys Met Pro Cys Phe Thr Thr Asp His Gln Met Ala Arg Lys
   1               5                   10                  15
   Cys Asp Asp Cys Cys Gly Gly Lys Gly Arg Gly Lys Cys Tyr Gly Pro
               20                  25                  30
   Gln Cys Leu Cys Arg
           35
<210> 4
   <211> 39
   <212> PRT
   <213> Leiurus quinquestriatus
<400> 4
   Tyr Ser Tyr Met Cys Met Pro Cys Phe Thr Thr Asp His Gln Met Ala
   1               5                   10                  15
   Arg Lys Cys Asp Asp Cys Cys Gly Gly Lys Gly Arg Gly Lys Cys Tyr
               20                  25                  30
   Gly Pro Gln Cys Leu Cys Arg
           35
<210> 5
   <211> 36
   <212> PRT
   <213> Artificial Sequence
<220>
   <223> chlorotoxin variant
<400> 5
   Met Cys Met Pro Cys Phe Thr Thr Asp His Gln Met Ala Arg Lys Cys
   1 5 10 15
   Asp Asp Cys Cys Gly Gly Lys Gly Arg Gly Lys Cys Phe Gly Pro Gln
   20 25 30
   Cys Leu Cys Arg
   35
<210> 6
   <211> 35
   <212> PRT
   <213> Artificial Sequence
<220>
   <223> chlorotoxin variant
<400> 6
   Arg Cys Lys Pro Cys Phe Thr Thr Asp Pro Gln Met Ser Lys Lys Cys
   1 5 10 15
   Ala Asp Cys Cys Gly Gly Lys Gly Lys Gly Lys Cys Tyr Gly Pro Gln
   20 25 30
   Cys Leu Cys
   35
<210> 7
   <211> 38
   <212> PRT
   <213> Artificial Sequence
<220>
   <223> chlorotoxin variant
<400> 7
   Arg Cys Ser Pro Cys Phe Thr Thr Asp Gln Gln Met Thr Lys Lys Cys
   1 5 10 15
   Tyr Asp Cys Cys Gly Gly Lys Gly Lys Gly Lys Cys Tyr Gly Pro Gln
   20 25 30
   Cys Ile Cys Ala Pro Tyr
   35
<210> 8
   <211> 7
   <212> PRT
   <213> Leiurus quinquestriatus
<220>
   <221> MISC_FEATURE
   <223> Derivative of chlorotoxin: amino acid residues 23-29
<400> 8
   Lys Gly Arg Gly Lys Ser Tyr
   1 5
<210> 9
   <211> 9
   <212> PRT
   <213> Artificial Sequence
<220>
   <223> Motif for chlorotoxin derivatives
<220>
   <221> variant
   <222> (3)..(3)
   <223> xaa can be Asp or Glu
<220>
   <221> variant
   <222> (4)..(4)
   <223> Xaa can be Ala, Arg, Asn, Asp, Cys, Gln, Glu, Gly, His, Ile, Leu, Lys, Met, Phe, Ser, Thr, Trp, Tyr, Val or Pro
<220>
   <221> variant
   <222> (5)..(5)
   <223> xaa can be Asn or Gln
<220>
   <221> variant
   <222> (7) .. (7)
   <223> xaa can be ser, Thr, or Ala
<220>
   <221> variant
   <222> (8)..(8)
   <223> xaa can be His, lys or Arg
<400> 9
   Thr Thr Xaa Xaa Xaa Met Xaa Xaa Lys
   1 5
<210> 10
   <211> 9
   <212> PRT
   <213> Leiurus quinquestriatus
<400> 10
   Thr Thr Asp His Gln Met Ala Arg Lys
   1 5
<210> 11
   <211> 9
   <212> PRT
   <213> Artificial Sequence
<220>
   <223> Chlorotoxin derivatives
<400> 11
   Thr Thr Asp Gln Gln Met Thr Lys Lys
   1 5
<210> 12
   <211> 9
   <212> PRT
   <213> Leiurus quinquestriatus hebraeus
<400> 12
   Thr Thr Asp Pro Gln Met Ser Lys Lys
   1 5
<210> 13
   <211> 7
   <212> PRT
   <213> Leiurus quinquestratus
<220>
   <221> MISC_FEATURE
   <223> Derivative of chlorotoxin: amino acid residues 8-14
<400> 13
   Thr Asp His Gln Met Ala Arg
   1 5
<210> 14
   <211> 9
   <212> PRT
   <213> Artificial Sequence
<220>
   <223> Chlorotoxin alpha peptide
<400> 14
   Thr Asp His Gln Met Ala Arg Lys Ser
   1 5
<210> 15
   <211> 9
   <212> PRT
   <213> Artificial Sequence
<220>
   <223> variant of chlorotoxin alpha peptide
<400> 15
   Thr Ala His Ala Met Ala Arg Lys Ser
   1 5
<210> 16
   <211> 36
   <212> PRT
   <213> Artificial Sequence
<220>
   <223> variant peptide of chlorotoxin
<400> 16
   Met Cys Met Pro Cys Phe Thr Thr Ala His Ala Met Ala Arg Lys Cys
   1 5 10 15
   Asp Asp Cys Cys Gly Gly Lys Gly Arg Cys Lys Cys Tyr Gly Pro Gln
   20 25 30
   Cys Leu Cys Arg
   35
<210> 17
   <211> 7
   <212> PRT
   <213> Artificial Sequence
<220>
   <223> chlorotoxin derivative STP-1
<400> 17
   Thr Asp Pro Gln Met Ser Arg
   1 5
<210> 18
   <211> 10
   <212> PRT
   <213> Artificial Sequence
<220>
   <223> Peptide 8 sequences
<400> 18
   Gly Gly Lys Gly Arg Gly Lys Ser Tyr Gly
   1 5 10
<210> 19
   <211> 9
   <212> PRT
   <213> Artificial Sequence
<220>
   <223> Peptide 8a Sequence
<400> 19
   Gly Lys Gly Arg Gly Lys Ser Tyr Gly
   1 5
<210> 20
   <211> 8
   <212> PRT
   <213> Artificial Sequence
<220>
   <223> Peptide 8b Sequence
<400> 20
   Lys Gly Arg Gly Lys Ser Tyr Gly
   1               5
<210> 21
   <211> 7
   <212> PRT
   <213> Artificial Sequence
<220>
   <223> Peptide 8c Sequence
<400> 21
   Gly Arg Gly Lys Ser Tyr Gly
   1 5
<210> 22
   <211> 10
   <212> PRT
   <213> Artificial Sequence
<220>
   <223> Peptide 21 Sequence
<400> 22
   Thr Thr Asp His Gln Met Ala Arg Lys Ser
   1 5 10
<210> 23
   <211> 8
   <212> PRT
   <213> Artificial Sequence
<220>
   <223> Peptide 21b Sequence
<400> 23
   Asp His Gln Met Ala Arg Lys Ser
   1 5
<210> 24
   <211> 7
   <212> PRT
   <213> Artificial Sequence
<220>
   <223> Peptide 21c Sequence
<400> 24
   His Gln Met Ala Arg Lys Ser
   1 5
<210> 25
   <211> 6
   <212> PRT
   <213> Artificial Sequence
<220>
   <223> Peptide 21d Sequence
<400> 25
   Gln Met Ala Arg Lys Ser
   1 5
<210> 26
   <211> 9
   <212> PRT
   <213> Artificial Sequence
<220>
   <223> Peptide 21a-A1 Sequence
<400> 26
   Gln Asp His Gln Met Ala Arg Lys Ser
   1 5
<210> 27
   <211> 9
   <212> PRT
   <213> Artificial Sequence
<220>
   <223> Peptide 21a-A2equence
<400> 27
   Thr Ala His Gln Met Ala Arg Lys Ser
   1 5
<210> 28
   <211> 9
   <212> PRT
   <213> Artificial Sequence
<220>
   <223> Peptide 21a-A3 Sequence
<400> 28
   Thr Asp Ala Gln Met Ala Arg Lys Ser
   1 5
<210> 29
   <211> 9
   <212> PRT
   <213> Artificial Sequence
<220>
   <223> Peptide 21a-A4 Sequence
<400> 29
   Thr Asp His Ala Met Ala Arg Lys Ser
   1 5
<210> 30
   <211> 9
   <212> PRT
   <213> Artificial Sequence
<220>
   <223> Peptide 21a-A5 Sequence
<400> 30
   Thr Asp His Gln Ala Ala Arg Lys Ser
   1 5
<210> 31
   <211> 9
   <212> PRT
   <213> Artificial Sequence
<220>
   <223> Peptide 21a-A7 Sequence
<400> 31
   Thr Asp His Gln Met Ala Ala Lys Ser
   1 5
<210> 32
   <211> 9
   <212> PRT
   <213> Artificial Sequence
<220>
   <223> Peptide 21a-A8 Sequence
<400> 32
   Thr Asp His Gln Met Ala Arg Ala Ser
   1 5
<210> 33
   <211> 9
   <212> PRT
   <213> Artificial Sequence
<220>
   <223> Peptide 21a-A9 Sequence
<400> 33
   Thr Asp His Gln Met Ala Arg Lys Ala
   1 5
<210> 34
   <211> 9
   <212> PRT
   <213> Mesobuthus tamulus sindicus
<220>
   <223> GenBank Accession No. P15229, small toxin
<400> 34
   Thr Thr Asp Gln Gln Met Ser Lys Lys
   1               5